(19) Europäisches Patentamt

European Patent Office

Office européen des brevets

(11) Numéro de publication : **0 401 445 B1**

(12) **FASCICULE DE BREVET EUROPEEN**

(45) Date de publication du fascicule du brevet :
**18.12.91 Bulletin 91/51**

(21) Numéro de dépôt : **89401487.7**

(22) Date de dépôt : **31.05.89**

(51) Int. Cl.⁵ : **A61K 35/02, A61K 33/00, A61K 33/42, // (A61K35/02, 33:42, 33:00, 31:19)**

(54) **Préparation aqueuse à base de sels minéraux et d'acide acétique pour la prévention et le traitement des maladies virales.**

Le dossier contient des informations techniques présentées postérieurement au dépôt de la demande et ne figurant pas dans le présent fascicule.

(43) Date de publication de la demande :
**12.12.90 Bulletin 90/50**

(45) Mention de la délivrance du brevet :
**18.12.91 Bulletin 91/51**

(84) Etats contractants désignés :
**ES GR**

(56) Documents cités :
**WO-A-89/06133**
**FR-A- 2 108 935**
**US-A- 3 676 553**
**"Rote Liste", 1961, page 54, Editio Cantor, Aulendorf/Württ, DE**
**UNLISTED DRUGS, vol. 31, no. 5, May 1979, page 71, Chatham, New Jersey, US**

(73) Titulaire : **Commin, Alix-Roland**
**2, place Gabriel Péri**
**F-94400 Vitry sur Seine (FR)**

(72) Inventeur : **Commin, Alix-Roland**
**2, place Gabriel Péri**
**F-94400 Vitry sur Seine (FR)**

Jouve, 18, rue Saint-Denis, 75001 PARIS

## Description

La présente invention propose, pour la prévention et le traitement des maladies virales, une solution aqueuse contenant, d'une part, de 15 à 20 g/l d'un mélange de sels formés à partir des cations sodium, magnésium, calcium, potassium, strontium et bore et des anions chlore, sulfate, carbonate et bicarbonate et, d'autre part, de 3 à 4% d'acide acétique, et son procédé de préparation.

Il est entendu que l'acide acétique peut donner lui aussi des sels avec les métaux alcalins et alcalino-terreux précités.

De préférence, la solution aqueuse renferme, en outre, des composés minéraux azotés choisis entre les nitrites, les nitrates et les sels ammoniacaux.

Avantageusement, la solution aqueuse renferme, en outre, des phosphates et de la silice.

Dans une forme d'exécution pratique de l'invention, la solution aqueuse est préparée à partir d'eau de mer et de vinaigre blanc, matières premières qui apportent les différents constituants voulus pour la solution.

Dans ce cas, la solution résultante est stérilisée par filtration à froid sur bougie poreuse.

La solution selon l'invention peut être administrée par voie parentérale, notamment par injection intraveineuse ou intramusculaire, auquel cas elle sera convenablement tamponnée et/ou diluée pour être isotonique au plasma sanguin.

La solution peut également être administrée sous forme de lavement intestinal.

Le médicament résultant convient à la prévention et au traitement des maladies virales, notamment du SIDA.

## Revendications

1. Procédé de préparation d'une solution aqueuse caractérisé en ce qu'il consiste à mélanger, d'une part, une solution aqueuse contenant de 15 à 20 g/l : d'un mélange de sels formés à partir des cations sodium, magnésium, calcium, potassium, strontium et bore et des anions chlore, sulfate, carbonate et bicarbonate et, d'autre part, 3 à 4% d'acide acétique.

2. Procédé selon la revendication 1, caractérisé en ce qu'il consiste à ajouter à la solution aqueuse des composés minéraux azotés choisis entre les nitrites, les nitrates et les sels ammoniacaux.

3. Procédé selon la revendication 1, caractérisé en ce qu'il consiste à ajouter des phosphates à la solution aqueuse.

4. Procédé selon la revendication 1, caractérisé en ce qu'il consiste à ajouter de la silice à la solution aqueuse.

5. Procédé selon l'une quelconque des revendications 1 à 4, caractérisé en ce que la solution aqueuse est préparée à partir d'eau de mer et de vinaigre blanc.

6. Procédé selon la revendication 5, caractérisé en ce que la solution est stérilisée par filtration à froid sur bougie poreuse.

7. Procédé selon l'une quelconque des revendications 1 à 5, caractérisé en ce qu'il consiste à convenablement tamponner et/ou diluer la solution aqueuse pour la rendre isotonique au plasma sanguin.

## Claims

1. Process for preparing a hydrous solution, characterized by the fact that it consists of mixing, firstly, a hydrous solution containing 15 to 20 g/l of a mixture of salts formed from the following cations : sodium, magnesium, calcium, potassium, strontium and boron, and from the following anions : chlorine, sulphates, carbonate and bicarbonate, and secondly, 3 to 4% of acetic acid.

2. Process according to claim 1, characterized by the fact that it consists of adding to the hydrous solution nitrogenous mineral compounds chosen from among the nitrates and ammonium chlorides.

3. Process according to claim 1, characterized by the fact that it consists of adding phosphates to the hydrous solution.

4. Process according to claim 1, characterized by the fact that it consists of adding silicon dioxide to the hydrous solution.

5. Process according to any of the above claims 1 to 4, characterized by the fact that the hydrous solution is prepared from sea water and white vinegar.

6. Process according to claim 5, characterized by the fact that the solution is sterilized by cold filtration on a porous candle.

7. Process according to any of the above claims 1 to 5, characterized by the fact that it consists of suitably buffering and/or diluting the hydrous solution to make it isotonic to blood plasma.

## Patentansprüche

1. Vorbereitungsverfahren einer wässrigen Lösung, gekennzeichnet durch eine Vermengung, einerseits einer 15 bis 20 g/l einer Salzmischung enthaltenden wässrigen Lösung — Salzmischung die im wesentlichen aus Natrium-, Magnesium-, Kalzium-, Kalium-, Strontium-und Bor-Kationen und aus Chlor-, Sulfat-, Karbonat- und Bikarbonat-Anionen gebildet ist — und andererseits mit 3-4% Essigsäure.

2. Verfahren gemäss Anspruch 1, gekennzeichnet durch Zusatz zur wässrigen Lösung von anorganischen Stickstoff-Verbindungen, ausgewählt unter Nitrit-, Nitrat- und Ammoniaksalzen.

3. Verfahren gemäss Anspruch 1, gekennzeichnet durch Zusatz von Phosphat zur wässrigen Lösung.

4. Verfahren gemäss Anspruch 1, gekennzeichnet durch den Zusatz von Kieselsäure zur wässrigen Lösung.

5. Verfahren gemäss irgenwelches der Ansprüche 1 bis 4 gekennzeichnet durch die Vorbereitung der wässrigen Lösung anhand von Meerwasser und weissem Essig.

6. Verfahren gemäss Anspruch 5, gekennzeichnet dadurch dass die Lösung sterilisiert wird durch kaltwegiges Filtrieren auf poriger Kerze.

7. Verfahren gemäss irgendwelches der Ansprüche 1 bis 5 gekennzeichnet dadurch, dass es darin besteht die wässrige Lösung passend zuzustöpseln und/oder die wässrige Lösung zu — verdünnen, um sie isotonisch mit Blutplasma zu gestalten.